(19) Europäisches Patentamt
European Patent Office
Office européen des brevets

(11) Publication number: **0 169 357 B1**

(12) **EUROPEAN PATENT SPECIFICATION**

(45) Date of publication of patent specification: **28.02.90**

(51) Int. Cl.$^5$: **C 10 M 125/00,** C 10 M 129/38, C 10 M 159/18 // (C10N10/02, 10:04, 10:06, 30:06)

(21) Application number: **85107141.5**

(22) Date of filing: **10.06.85**

(60) **Divisional application 89104779.7 filed on 10/06/85.**

(54) **Crystalline aluminates as lubricant additives.**

(30) Priority: **11.06.84 US 619442**
**11.06.84 US 619427**

(43) Date of publication of application:
**29.01.86 Bulletin 86/05**

(45) Publication of the grant of the patent:
**28.02.90 Bulletin 90/09**

(84) Designated Contracting States:
**BE CH DE FR GB IT LI NL SE**

(56) References cited:
**EP-A-0 103 034**
**US-A-4 348 297**
**US-A-4 392 980**

(73) Proprietor: **THE DOW CHEMICAL COMPANY**
**2030 Dow Center Abbott Road P.O. Box 1967**
**Midland Michigan 48640-1967 (US)**

(72) Inventor: **Kar, Kishore K.**
**4512 North Saginaw Road**
**Midland Michigan 48640 (US)**
Inventor: **Burba III, John L.**
**168 Dallas**
**Angleton Texas 77515 (US)**
Inventor: **Bauman, William C.**
**2900 Braley Court**
**Midland Michigan 48640 (US)**

(74) Representative: **Casalonga, Axel et al**
**BUREAU D.A. CASALONGA - JOSSE**
**Morassistrasse 8**
**D-8000 München 5 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 169 357 B1

## Description

This invention concerns a process for improving the anti-friction properties of lubrication fluids which are subjected to intensive shearing or grinding forces. The present invention also concerns crystalline lithium aluminate compositions useful as additives for lubrication fluids.

Crystalline aluminate compositions conforming generally to the empirical formula $Li^+(RCOO^-)\cdot 2Al(OH)_3\cdot nH_2O$, where $RCOO^-$ represents an organic acid anion, and $nH_2O$ represents any waters of hydration, are disclosed, inter alia, in U.S. 4,348,295, U.S. 4,348,296, and U.S. 4,348,297.

The following patents are believed to be representative of some of the prior art regarding extreme pressure lubricant additives or lithium aluminate compounds: U.S. 2,621,159; U.S. 3,001,939; U.S. 3,093,584; U.S. 3,318,808; U.S. 3,565,802; U.S. 3,909,426; U.S. 3,984,599; U.S. 3,997,454; U.S. 4,116,856; U.S. 4,116,858; U.S. 4,159,311; U.S. 4,221,767; U.S. 4,293,430; U.S. 4,347,327; U.S. 4,321,065; U.S. 4,376,100; and U.S. 4,381,349.

Also disclosed are crystalline $LiX\cdot 2Al(OH)_3\cdot nH_2O$ compounds and derivatives thereof, e.g., where the X anion represents OH, halide, halo acid, inorganic acid, organic acid and others. The compounds are referred to generally as "lithium aluminates" and are prepared, principally, by reacting lithium salts with hydrous alumina and forming crystalline $LiX\cdot 2Al(OH)_3\cdot nH_2O$ which in some cases are of the "two-layer" variety and in some cases of the "three-layer" variety, depending on the particular method or materials employed. Methods for preparing these known crystalline lithium aluminates, of the $LiX\cdot 2Al(OH)_3\cdot nH_2O$ formula, both 2-layer and 3-layer varieties, and anion exchanges or replacements in the crystals, are disclosed in the patents identified above.

In the prior art the principles of the following tests, or variations thereof, are usually followed:

*ASTM D—2509* "Standard Method for Measurement of Extreme Pressure Properties of Lubricating Grease (Timken Method)".

*ASTM D—2782* "Standard Method for Measurement of Extreme Pressure Properties of Lubricating Fluids (Timken Method)".

*ASTM D2783* "Standard Method for Measurement of Extreme Pressure Properties of Lubricating Fluids (Four-Ball Method)".

In this present disclosure the expression "lithium stearate aluminate" (also "LSA") is a crystalline compound of the formula, $Li^+(RCOO^-)\cdot 2Al(OH)_3\cdot nH_2O$ where $RCOO^-$ is the negative-valent carboxylate radical of stearic acid. It is prepared in accordance with the procedure disclosed in U.S. 4,348,295 or U.S. 4,348,297 whereby crystalline $LiOH\cdot 2Al(OH)_3\cdot nH_2O$, material is reacted with stearic acid, thereby replacing the $OH^-$ (attached to the Li) with $RCOO^-$.

Lithium stearate aluminates (LSA) are organic-inorganic (60:40) hybrid crystalline materials conforming substantially to the empirical formula $LiX\cdot 2Al(OH)_3\cdot nH_2O$ where X is an anion (stearate) and $nH_2O$ represents water of hydration. These are 2- or 3-layer unit cell structures. The particle size is usually from 150 Å to 5000 Å. TGA studies have shown that it decomposes at 300°C. X-ray diffraction and SEM analyses have revealed its platelet structure. These LSA compounds also include the compounds of formula I, II, III or IV as defined below.

Industrial oils and lubricating fluids frequently require friction reducers for energy saving and antiwear/ extreme pressure additives to extend their functional range. Tribological research has now been conducted to evaluate the lubrication performance of lithium stearate aluminate as an additive in lubricants. The place of LSA within the lubricant industry is found, e.g., in its application as an extreme pressure (EP), anti-wear and friction-reducing additive.

Extreme pressure (EP) and anti-wear additives are used mainly to improve the performance of lubricants. As a class, such substances produce a physical or chemical effect on the surfaces of the friction pair, thus leading to a reduction in wear rate under conditions of mixed or boundary lubrication and an increase in the seizure load. Such additives are called extreme pressure and antiwear additives.

The principal effect of an EP additive occurs under heavy loads when, in addition to high temperatures, the metallic surface is activated mechanically (tribochemical effect). It is known that freshly worn surfaces are a source of electrons which are capable of initiating several reactions which would otherwise not occur. In some cases, the additive or additives present in the lubricant, in contact with the frictional surface at high temperatures, undergo polymerization or reaction with one another leading to the formation of a solid compound on the surface. The polymer layer formed by the insitu polymerization at high temperature, affords protection of the metallic surfaces against corrosion, serving as an antioxidant.

It is well known that some extreme pressure additives such as chlorinated paraffins, sulfo-chlorinated oils, or zinc dithiophosphates react with metallic surfaces during the frictional process. The reaction layer may improve the frictional properties of the metallic surfaces if it is a low shear strength compound or may simply prevent direct contact between the surfaces and the formation of junctions. Reaction between the metallic surface and the additive may also reduce adhesion. At the point of contact between the surfaces where the temperature is high, the additive prevents the formation of an adhesion bridge by reacting with the metallic surface. As asperities are the initial contact points, the process may lead to polishing of the surface (chemical polishing). Some of the EP additives (e.g. Zn-dithiophosphates) also possess antioxidant characteristics having two or more active elements in their molecules.

If the effectiveness of EP and antiwear additives is due to the reaction layer formed on the metallic

surfaces in contact, additive reactivity should be controlled, that is, the reaction between the metallic surface and the additive should take place only on the friction surface. Excessive reactivity may cause corrosion while low reactivity may not permit the formation and preservation of a protective layer on the surfaces of the friction pair as the existence of the layer in the contact area is the result of an equilibrium between the formation and wear processes. For this reason, chlorinated paraffin oils have limited use as antiwear-extreme pressure additive. It is reported that the presence of chlorinated-type additives in metalworking fluids often initiate corrosion of the machines over a long period of idleness. Some chlorine and sulfur base additives are reported to irritate skin or produce foul odors.

Long chain lubes such as the esters of fatty acids, aliphatic alcohols, and amines are used as friction reducers and antiwear additives in lubricants. A characteristic of their effectiveness is determined by the stability of the layer on the frictional surface. Usually at relatively low temperatures up to 150°C, the layer is desorbed, losing its effectiveness. The melting points of most of the long chain lubes (metallic soaps) are less than 150°C. For effective lubrication above 150°C, surface films withstanding higher temperatures must be used. Some lamellar solids, such as graphite and molybdenum disulfide ($MoS_2$), having low intrinsic shear strength because of their layer lattice crystal structure, are used as solid lubricants. The lubrication effectiveness is attributed to the formation of an adhering film rather than a reactive film. Solid lubricants are well known as friction reducers. Crystalline lithium aluminates (stearic anion) decompose at 300°C, whereas $MoS_2$ and graphite melt at 400°C and 500°C.

The particle size usually affects the lubricating properties of the suspension. Experimental work carried out on a four-ball tester has shown that if colloidal suspensions are used, the optimum mean diameter of the $MoS_2$ particle is around 25,000 Å. A complex interdependence exists between particle size and the antiwear characteristics of the suspension. Under light loads particle size has no effect, while under heavy loads larger grain sizes usually result in increased wear.

In one embodiment, the present invention concerns a process for improving the anti-friction properties of lubrication fluids subjected to intensive shearing or grinding forces, which comprises uniformly dispersing in said fluid, as small particles, at least one crystalline aluminate conforming substantially to the formula:

$$\text{I. } (LiA_x)_y \cdot 2Al(OH)_3 \cdot nH_2O$$

$$\text{II. } Li(R)_r^v \cdot 2Al(OH)_3 \cdot nH_2O, \text{ or}$$

$$\text{III. } M_m(R)_r^v(D)_z^w \cdot 2Al(OH)_3 \cdot nH_2O$$

wherein A is one or more anions and/or negative-valance radicals or mixtures thereof;

wherein, in formula I, x is a quantity of A ions or radicals sufficient to substantially satisfy the valence requirements of Li;

wherein n is zero or the number of waters of hydration;

wherein y is a numerical value at least sufficient to maintain the crystalline structure;

wherein R is an anion of a monocarboxylic acid or dicarboxylic acid of $C_6$—$C_{22}$, including those which are $OH^-$ substituted;

wherein r is greater than zero and represents the number of R ions in the molecule;

wherein v is the valence of R, being monovalent or divalent;

wherein M is divalent Zn or Ca cations;

wherein D represents inorganic anions of valence 1-3, represented by w;

wherein z is equal to or greater than zero and represents the number of D anions;

wherein, in formula II, vr represents an amount of R anions to substantially satisfy the valence requirements of Li;

wherein, in formula III, $r<z$ and $(vr + wz)$ represents an amount of combined anions, R and D, to substantially satisfy the valence requirements of M;

with m representing the number of divalent M cations and having a numerical value in the range of 1 to 4.

The preferred composition of the present invention comprises a substantially uniform mixture of a lubrication fluid and at least one compound of formula I as defined above.

An especially preferred composition comprises a mixture of a lubrication fluid and a crystalline lithium aluminate of the formula

$$\text{IV. } (Li(OH)_{1-gp}E_p^{-g})_y \cdot 2Al(OH)_3 \cdot nH_2O$$

wherein E is at least one anion or negative-valence radical other than $OH^-$, with a valence of $-g$;

wherein p is greater than or equal to zero and is less than or equal to unity; and

wherein y and n are defined as above.

Brief Description of Drawings

Figure 1 is an illustration, not to scale, for use as a visual aid in describing wear rates in load-capacity tests.

Figure 2 illustrates curves for wear scar diameter vs. increasing load for an oil and for the oil with LSA of formula II added thereto.

Figure 3 illustrates the improvement in wear coefficient obtained by adding LSA of formula II to an oil.

The aluminate crystal of formula I or II may be of the two-layer or three-layer variety or may be a mixture of the two varieties.

As shown in the above patents, hydrous alumina, represented by the formula $Al(OH)_3$, may be suspended in an ion exchange resin and then reacted with aqueous LiOH at elevated temperature to form crystalline $LiOH \cdot 2Al(OH)_3$.

It is understood, of course, that the so-formed crystalline aluminates, being in contact with water, have waters of hydration attached.

The above patents also disclose that the crystalline $LiOH \cdot 2Al(OH)_3$ is beneficially converted to $LiX \cdot 2Al(OH)_3$, where X is a halogen, i.e. Cl, Br, or I.

It is also disclosed that the crystalline $LiOH \cdot 2Al(OH)_3$, whether supported within or on a substrate, or prepared in the absence of a substrate, is beneficially converted to other lithium aluminates by reactions which replace the OH radicals with other anions or radicals.

Substrates in addition to ion exchange resins contemplated in accordance with the present invention include, e.g., inorganic substrates (which are substantially inert to the reactions involved in preparing the $(LiA_x)_y \cdot 2Al(OH)_3 \cdot nH_2O$), inert organic or inert polymeric substrates, and inert metallic substrates.

The "neat" preparations of the subject compounds, i.e. in the absence of a substrate, are also contemplated according to the present invention and usually allow larger aggregates or stacks of the crystals in the crystalline structure.

The anions contemplated by A in the above formula (including halide and hydroxyl) include the anions of soluble inorganic acids, mineral acids, organic acids, or anions of the salts of such acids.

The anions of inorganic acids and mineral acids include, for example, $SO_4^{--}$, $HCO_3^-$, $BO_22^-$, $H_2PO_4^-$, $HPO_4^{--}$, $ClO_4^-$, $HCrO_4^-$, $NO_3^-$, $SO_3^{--}$, $HSO_3^-$, $NO_2^-$, $H_2AsO_4^-$, $HAsO_4^{--}F^-$, $HS^-$, $ClO_3^-$, $H_2PO_3^-$, $HPO_3^{---}$, $H_3P_2O_7^-$, $MnO_4^-$, $H_2P_3O_7^{--}$, $HP_2O_7^{---}$, $NH_2SO_3^-$, $H_2PO_4^-$, $PO_4^{---}$, and the like.

The anions of organic acids may be derived, for example, from monobasic acids (RCOOH), dibasic acids (HOOC—COOH or HOOC—R—COOH), tribasic acids (HOOC—R(COOH)—COOH) where R is a substituted or unsubstituted hydrocarbon moiety, and other multibasic organic acids, such as ethylenediamine tetraacetic acid, acrylic acid polymers and copolymers, pyromellitic acid, and the like. Examples of monobasic acids are, for instance, formic acid, acetic acid, chloroacetic acid, dichloroacetic acid, trichloroacetic acid, acrylic acid, methacrylic acid, crotonic acid, butyric acid, propionic acid, tartaric acid, hexanoic acid, fatty acids (such as stearic acid), aromatic and cyclic acids (such as benzoic, toluic, salicylic, gallic, cinnamic acid), and the like. Examples of dibasic acids are, for instance, oxalic acid, malonic acid, fumaric acid, malic acid, maleic acid, succinic acid, terephthalic acid, pimelic acid, and the like. Citric acid is an example of a tribasic acid, $HOOCCH_2C(OH)(COOH)CH_2COOH$. Quadribasic acids, such as pyromellitic acid, are also contemplated by the present invention. Hydroxy carboxylic acids, such as glycollic acid, lactic acid, tartaric acid, and malic acid are also contemplated by the present invention. Organic radicals with inorganic substituents, such as $CH_2SO_3^-$, $CH_3PO_3^{--}$, and $C_6H_{11}SO_3^-$ are also contemplated by the present invention.

Crystalline lithium aluminates conforming to the formula $Li(RCO\overline{O})_y \cdot 2Al(OH)_3O_2 \cdot nH_2O$, wherein $RCO\overline{O}$ is a fatty acid anion, y is the number of Li atoms for each 2 Al atoms and n is zero or a positive amount of waters of hydration, are found to be useful as additives to organic fluids as thickeners, as viscosity control agents, as compatabilizers, and/or as dispersing agents. These aluminates are especially useful as additives to silicone oils and lubricants, synthetic oils and lubricants, organics, and hydrocarbons, most especially aliphalic hydrocarbons such as mineral oils, petroleum oils, motor oils, diesel oils, vegetable oils, and the like. In those aluminates wherein the fatty acid anion is derived from fatty acids having 10 or more carbon atoms in the aliphatic carbon chain, or branched-chains, the aluminate is, itself, a useful grease or lubricant.

As used within the purview of this disclosure, the $Li(RCOO)_y \cdot 2Al(OH)_3 \cdot nH_2O$ compounds include the 2-layer and 3-layer varieties such as disclosed in U.S. 4,348,295 and U.S. 4,348,296. When written as $Li(RCOO)_y \cdot 2Al(OH)_3 \cdot nH_2O$, the subscript y is used (as in U.S. 4,348,297) to indicate the number of Li atoms per each 2 Al atoms; the value of y is preferably 1.0, but may be from 0.5 to 2, depending on how the crystals are prepared and on how much (if any) Li values have been leached or exchanged out of the crystals. In some cases the value of n can be virtually zero, indicating that waters of hydration are essentially absent, but in the absence of an intensive drying procedure, the value of n is usually in the range of 0 to 6.

The $RCOO^-$ anion in the lithium aluminate crystal may be any fatty acid wherein R is an aliphatic carbon chain or branched-chain, having one or more carbon atoms. In those instances wherein it is desired that the aluminate compound be employed, e.g. as a thickener, a gelling agent, a processing aid, a dispersing agent, and the like, in various oils, water dispersions, organic fluids, or as a grease or lubricant itself, it is preferred that the $RCOO^-$ anion contain more than 8 carbon atoms, more preferably 12 or more carbon atoms, most preferably 14 to 22 carbon atoms.

In formula II, R is a monocarboxylic acid or dicarboxylic acid of $C_6$—$C_{22}$ acids, such as hydroxy-stearic acid, adipic acid, decanoic acid, lauric acid, stearic acid, behenic acid, and others given above.

Compounds of formula III in the above are prepared from layered crystalline compounds of the formula $M_mD_z^w \cdot 2Al(OH)_3$, where M is Ca or Zn, where D represents inorganic anions of valence (w) of 1 to 3, where z represents a quantity of D ions to substantially satisfy the valence requirements of M, and where m represents an amount in the range of 1—4. This is done by mixing it with the desired carboxylic acid in aqueous or alcoholic medium thereby replacing some or all of the inorganic ions with R ions.

The symbol "D" in Formula III represents an inorganic anion of valence 1 to 3 which, when combined with M cations forms a salt, e.g., sulfate, hydroxide, phosphate, hydrogen phosphate, chloride, bromide, carbonate, nitrate, or bicarbonate.

The lubrication fluid may be an oil or grease comprising an aliphatic, hydrocarbon, organic, or silicone material. The said lubrication fluid may be emulsified or dispersed in an aqueous carrier. A silicone oil or grease may be dispersed in an aqueous carrier or in an aliphatic, organic, or hydrocarbon oil or grease.

The aluminate compound may be emulsified or dispersed in the lubrication fluid by any convenient means, such as by use of an agitator, a recycle pump, a sonic mixer, or an in-line static mixer; a dispersing aid or additive is usually beneficial.

The amount of the aluminate compound which is used in the lubrication fluid is usually from 0.1 percent by weight to 10 percent by weight. Preferably, the amount of aluminate compound in the lubrication fluid is 0.2 percent to 2.0 percent by weight. The particles of the aluminate compound dispersed in the lubrication fluid are of a size in the range of 150 to 5000Å.

The lithium aluminates of the present invention can also be added to polymers, waxes and paraffins which can be sufficiently fluidized at a temperature, generally, less than about 250—300°C to permit adequate mixing with the aluminate. These mixtures are useful, e.g., as lubricants, mold release agents, fire-retarding additives, and polymer additives. These lithium aluminates also serve to reinforce polymers or resins or other solidified materials to which they are added.

Of the fatty acids which are the source of the $RCOO^-$ anions of the crystalline lithium aluminates of the present invention, those which have from 1 to 8 carbon atoms in their molecule are at least partially soluble in water at 20°C, but those with 9 or more carbon atoms in their molecule are practically insoluble in water at 20°C. Thus, in preparing $Li(RCOO^-) \cdot 2Al(OH)_3 \cdot nH_2O$ by the reaction of $CH_3(CH_2)_xCOOH$ (x is at least 7) with $LiOH \cdot 2Al(OH)_3 \cdot nH_2O$, a solvent or reaction medium other than water may be used. A convenient solvent or carrier is alcohol, such as isopropanol, though other solvents or carriers for the fatty acid may be used, such as hexane, toluene, oils (e.g. mineral oils), ethers, halocarbons, silicone fluids, and the like. The fatty acid itself, so long as it is at a temperature at which it is molten, can serve as its own reaction medium. For example, nonylic acid melts at about 12.5°C and stearic acid melts at about 69°C.

A modicum of success is achieved by carrying out the long chain $RCOO^-$ intercalations in a water carrier if the fatty acid (molten or solid) is finely dispersed in the water, or a solution of the fatty acid is finely dispersed in water, and conducting the reaction with the crystalline lithium aluminate, preferably with stirring and at elevated temperature.

Of particular interest are $Li(RCOO^-)_y \cdot 2Al(OH)_3 \cdot nH_2O$ crystals wherein the $RCOO^-$ radical is oleic acid anion, stearic acid anion, linoleic acid anion, linolenic acid anion, benzoic acid anion, and the like. These aluminates are thin platey structures which are substantially thermally stable to temperatures of 300°—400°C. Some of them have about the same consistency as candle wax or soap and are useful as lubricants or greases at moderately high temperatures where many known hydrocarbon greases may lose their viscosity to the extent of being virtually ineffective as lubricants. Furthermore, these aluminates exhibit an ability to beneficially thicken hydrocarbon oils. For instance, 3-layer lithium oleate aluminate and 3-layer lithium stearate aluminate are successfully dispersed in mineral oil, motor oil, and diesel oil by adding about 30 g. of the material to 300 g. of the oil by the action of an ultrasonic disperser operated about 12 minutes at a temperature of 100°C. Stable water-in-oil dispersions can be prepared by dispersing lithium stearate aluminate in oils, e.g. diesel oil, motor oil, mineral oil, hydraulic fluids, and the like, which contain, e.g. 10 percent by volume $H_2O$.

The crystalline lithium aluminates of the present invention are prepared according to the following general procedure.

Crystalline or amorphous hydrous alumina, denoted as $Al(OH)_3$, is reacted at elevated temperature to form crystalline $LiOH \cdot 2Al(OH)_3 \cdot nH_2O$ in an aqueous medium. The beginning hydrous alumina may be unsupported by a substrate, or may be supported on a substrate, or may be dispersed or suspended within a porous substrate. The reaction between the hydrous alumina and the LiOH may take place at room temperature but to assure that the reaction is substantially completed within a reasonable length of time, an elevated temperature of at least 50°C, preferably at least 75°C, should be used. The amount of LiOH should not be in such excess that the aluminate is caused to precipitate outside the pores. The aqueous media may contain other ingredients and, if they are substantially inert or do not interfere with the desired reaction, are permissible. Insoluble, substantially inert particles may be present in the aqueous medium and may serve as a substrate for the $LiOH \cdot 2Al(OH)_3$ as it is formed. Choice of a substrate (if used) is dependent, of course, on the intended use of, or application of, the crystalline $LiOH \cdot 2Al(OH)_3 \cdot nH_2O$.

The present invention is not limited to a particular means for providing the beginning hydrous alumina for reaction with the LiOH. For example, the pores of a substrate may be substantially filled with $Al(OH)_3$ by growing seeds of $Al(OH)_3$ in the pores from an aqueous solution of sodium aluminate.

The crystalline $LiOH \cdot 2Al(OH)_3 \cdot nH_2O$ is then reacted in aqueous medium with anions or negative-valence radicals (A as hereinbefore described) having a valence of 1, 2, or 3 or more to form the $(LiA_x)_y \cdot 2Al(OH)_3 \cdot nH_2O$ compounds of the present invention. A monovalent anion or radical yields $(LiA)_y \cdot 2Al(OH)_3 \cdot nH_2O$. A divalent anion or radical yields $(LiA_{1/2})_y \cdot 2Al(OH)_3 \cdot nH_2O$. A trivalent anion or radical yields $(LiA_{1/3})_y \cdot 2Al(OH)_3 \cdot nH_2O$. Radicals of valence greater than 3 are similarly stoichiometrically balanced. The value of y is normally 1, but the actual value of y may vary over the range of 0.5 to 2.0, especially 0.5 to 1.2.

The so-prepared lithium aluminates are useful in selectively recovering $Li^+$ ions from solution if the amount of $LiA_x$ in the aluminate structure is first reduced to a lower concentration (but not completely removed), leaving space in the crystal for taking up $LiA_x$ salt until the crystal is once again "loaded" with $LiA_x$ salt. The A and x are as hereinbefore described.

The so-prepared lithium aluminates are also useful in exchanging of anions in aqueous solution, where an anion in solution replaces the anion in the crystal. For instance, where the A anion is the ascorbate radical of ascorbic acid (Vitamin C), the ascorbate anion is replaced by Cl in aqueous HCl, thereby providing ascorbic acid in the aqueous medium. The anion of ascorbic acid (a lactone) is formed by a keto-to-enol shift. The exchange of anions is also possible in non-aqueous systems, such as an alcohol, or in molten polymers or paraffins, such as polyethylene, polypropylene, polyvinylidene chloride, and the like.

It is well known that catalytic systems based on zeolite crystals are quite sensitive to inter crystalline spacing. The lithium aluminates used in the present invention provide an array of catalysts wherein the interplane or spacing of the crystalline aluminate structure is varied according to the size of the anion in the lithium aluminate.

The novel compositions of the present invention are prepared by substantially uniform mixing of an lubrication fluid and at least one crystalline lithium aluminate compound of the formula

$$\text{I. } (LiA_x)_y \cdot 2Al(OH)_3 \cdot nH_2O$$

wherein A, x, y and n are as hereinbefore defined.

The lubrication fluid contemplated in the composition of the present invention includes a hydrocarbon and a silicone.

The hydrocarbon contemplated by the lubrication fluid of the present invention includes aliphatic, paraffinic, bicyclic, alicyclic, aromatic, alkane, alkene, arylene, isoalkylene, isoalkane, or isoalkene hydrocarbons, including those which are substituted or unsubstituted, and including those which contain heteroatoms of the group consisting of N, S, O, Si, P, F, Cl, Br, and I.

The lubrication fluid may comprise a hydrocarbon wax, a paraffin, an olefin polymer, an olefin copolymer, a vinyl polymer, a vinyl copolymer, a polycarbonate, a polyalkyleneimine, a polyether, an epoxy, a polyurethane, a polysulfone, a polysiloxane, a polyterpene, a polyfluorocarbon, a polyimide, a silicone resin, a polyamide, a polyalkyleneoxide, or a polyarcylate.

The lubricant may be used as such or dissolved in a suitable solvent. The lubricant may itself be a liquid at ambient temperatures and pressure and may contain at least a trace amount of halogen present therein.

The mixing of the lubrication fluid and the crystalline lithium aluminate compound can be done by any known mixing means such as by the use of an agitator, a recycle pump, a sonic mixer, an in-line mixer and the like.

The embodiments of tests and examples which follow are only to illustrate the invention

Load-Capacity Tests

Load-capacity tests are generally used to determine the ability of a lubricant to prevent severe adhesive wear or seizure; they consist of wear tests run at different and progressively high loads in a 4-ball wear test apparatus. The diameter of wear scars produced on the balls at a given load are measured. With reference to Fig. 1, it is illustrated that the wear scar diameter may gradually increase (line BC) as the load (X) is increased until a transition region (line CD on Fig. 1) is reached where the wear rate is substantially increased and the slope of the line is steep. Further increases in pressure result in an incipient seizure region (line DE of Fig. 1) until contact junction temperatures are elevated so high that the lubricating film is rendered ineffective (at point E) and massive adhesion then leads to welding.

The 4-ball wear test apparatus is a widely used means for evaluating lubricants and lubricant additives under heavy loads, i.e., extreme pressure. For the tests disclosed here, a test device, sold under the tradename "Falex Model No. 6 Friction and Wear-Test Machine", with 4-ball test adapter, was employed using four 0.5-inch bearing balls (AISI—E—52100, grade 25) with roundness specification of $6.35 \times 10^{-4}$ mm. In the test there are three balls (each touching the other two) in a cup which rests in a stationary specimen holder. The fourth ball is placed on top of (and in respective contact with) the three balls; this fourth ball is held in place by an upper specimen holder which affixes to a vertically-disposed spindle. The lower specimen holder is supported by a lower shaft which is equipped with means for measuring wear rate and torque. Thermocouples inserted into wells in the wall of the four-ball cup are used, as needed, in measuring the temperature during the test.

Friction and Wear Tests

Bench wear tests are often used to evaluate anti-wear and extreme pressure characteristics of

lubricants. In this study, the Falex four-ball wear test method for evaluating the EP/anti-wear characteristics of paraffinic (e.g., Rubrex-100 oil), paraffinic-naphthalhenic blend (e.g. Flowrex-200 oil) oils with and without additives were examined. The Falex friction and wear tester was chosen because of its availability and because it facilitates the acquiring of torque versus wear cycle data on real time basis for determination of coefficient of friction or wear coefficient. The four-ball test is used extensively in industry for wear testing. The test is easy to conduct, well controlled and uniform test specimens are available at low cost. In wear testing, the specimens basically undergo a destructive evaluation process. In the four-ball test, bearing balls (AISI—E—52100, grade 25) with roundness specification of 6.35 × $10^{-4}$ mm are readily available. In experiments employing the four-ball machine, wear is generally determined by measuring the average scar diameter.

Four-Ball Test Conditions

The extreme pressure and the anti-wear lubrication properties of the experimental additive aluminate of formula II or III were evaluated by four-ball tests. Test loads were varied from 22.6 to 90.4 Kgf (50—200 lbs). These correspond to an approximate Hertzian contact pressure of 1750—7000 N/m$^2$ (25,000—100,000 psi). The test speed was 1000 RPM and each test ran for 50 minutes. Approximately 15—20 cc of fluid was used for each test. Before each test is conducted, the balls, top ball chuck and sample container are thoroughly washed with reagent grade hexane, toluene and acetone. The specimen holder and balls were dried at 75°C, then cooled to room temperature prior to the test run. The upper spindle was rinsed with toluene. After the tests were over, optical microscope pictures were taken and the scar diameters were measured from these pictures. During each test run, the torques, as a function of the wear cycles, were monitored on a real time data acquisition basis for data analyses.

In accordance with standard procedures known in the art, the following experiments were carried out:

Example 1

The effect of various concentrations of LSA of formula II in a commercially available lubricant sold under the tradename "Rubrex" oil is tested in the 4-ball tester at 100 lb. load for 50 minutes and the following data (Table I) indicate the effect on wear rate.

TABLE I

| Concentration of LSA in Rubrex oil (wt.%) | Average wear scar diameter (mm) |
|---|---|
| 0 | 0.96 |
| 0.24 | 0.73 |
| 0.5 | 0.67 |
| 1.0 | 0.63 |
| 1.5 | 0.62 |

Example 2

The effect on wear scar diameter vs. LSA of formula II concentration of different particle sizes (viz. 150 Å and 4000 Å) on a commercially available lubricant sold under the tradename "Flowrex" oil is tested as in Example 1 except that the load is 150 lb. and the duration is 50 minutes. Table II illustrates the results.

TABLE II

| Concentration of LSA in Flowrex oil, %/Å | Average wear scar diameter (mm) |
|---|---|
| 0/0 | 0.83 |
| 0.25/150 | 0.80 |
| 0.25/4000 | 0.67 |
| 0.5/150 | 0.65 |
| 0.5/4000 | 0.64 |
| 1.0/150 | 0.66 |
| 1.0/4000 | 0.57 |
| 1.5/150 | 0.66 |
| 1.5/4000 | 0.56 |

Example 3

Various amounts of LSA of formula II in Rubrex oil are found to reduce the coefficient of friction of the oil. Data are in Table III.

TABLE III

| Concentration of LSA in Rubrex oil (wt.%) | Torque inch/lbs | Friction Reduction (%) |
|---|---|---|
| 0 | 2.2 | — |
| 0.25 | 2.0 | 9 |
| 0.50 | 1.6 | 27 |
| 1.0 | 1.7 | 23 |
| 1.5 | 1.7 | 23 |

Example 4

Various amounts of LSA of formula II in Flowrex oil are found to reduce the coefficient of friction of the oil. Data are in Table IV.

TABLE IV

| Concentration of LSA in Flowrex oil (wt.%) | Torque, inch/lbs | Friction Reduction (%) |
|---|---|---|
| 0 | 2.9 | — |
| 0.25 | 2.6 | 10 |
| 0.50 | 2.8 | 3 |
| 1.0 | 2.25 | 22 |
| 1.5 | 2.2 | 24 |

Example 5

The average scar diameter of Flowrex-200 oil (a naphthenic-paraffinic oil) is measured, and compared with portions of the same oil containing, respectively, 1 percent by weight of lithium stearate and 1 percent by wt. of LSA of formula II. With reference to Fig. 1 attached hereto, it is found that the values of X at points B, C and D are as follows, with average scar diameter, in mm, shown in parentheses.

TABLE V

| Sample | Load at Point B (kg) | Load at Point C (kg) | Load at Point D (kg) |
|---|---|---|---|
| Flowrex-200 oil* | 22 (.61 mm) | 59 (.60 mm) | 68 (.80 mm) |
| Flowrex-200 oil* containing 1% by wt. stearic acid | 22 (.62 mm) | 45 (.57 mm) | 68 (.82 mm) |
| Flowrex-200 oil containing 1% by wt. LSA of formula I | 22 (.52 mm) | 77 (.58 mm) | 90 (.80 mm) |

*for comparison with present invention.

There is found little change in the average scar diameter of each of the above three samples between points B and C, but additional increased loads, greater than at point C, gives a steep climb in the curve of the average scar diameter, indicating that the transition range has been reached.

Example 6

Fig. 2 attached hereto illustrates the wear scar curves vs. increasing loads for a paraffinic oil (i.e. Rubrex-100 oil) and for the same oil with 1 percent by weight of LSA of formula II added thereto. The LSA of formula II not only extends the load carrying capacity (from about 52 Kgf to about 75 Kgf) but also reduces the scar diameter at a given load.

Example 7

Fig. 3 attached hereto illustrates the wear coefficient v. increasing loads for Rubrex oil and for Rubrex oil with 1 percent by weight of LSA of formula II additive.

Example 8

Tests were made using various compounds which conform essentially to formula II or III as described above.

The following Table VI shows data obtained using 1 percent by weight of the additive of formula II or III in a lubrication oil, Flowrex-200 (Mobil Oil), which exhibits a viscosity of 40 cst at 40°C. The tests were made using a Four-Ball method according to ASTM D—2266 under a constant force of 20 kg at 1800 rpm for 1 hour at 130°F (54.4°C).

TABLE VI

| Flowrex-200 Plus 1% Additive | No. of c's in Fatty Acid | Wear Scar Diameter (mm)* |
|---|---|---|
| Control, no additive | — | 0.61 |
| Lithium Stearate Aluminate (LSA) | 18 | 0.55 |
| Lithium Laurate Aluminate | 12 | 0.65 |
| Calcium Stearate Aluminate | 18 | 0.59 |
| Lithium Decanoate Aluminate | 10 | 0.66 |
| Zinc Stearate Aluminate | 18 | 0.54 |
| Lithium Behenate Aluminate | 22 | 0.73 |

* In this test a scar diameter of 0.8 mm or less is considered passing.

Example 9

The break point (i.e. point C at load X as illustrated in Fig. 1) of various embodiments are shown in Table VII, all samples using 1 percent additive of formula II or III in the Flowrex-200 as in Example 8, but using increases in the force applied.

TABLE VII

| Oil Plus 1% Additive | No. of c's in Fatty Acid | Average Wear Scar Diameter (mm)* | Break Point lbs/kg |
|---|---|---|---|
| Control, no additive | — | 0.63 | 124/56.3 |
| Lithium Adipate Aluminate | 6 | 0.65 | 131/59.5 |
| Lithium Decanoate Aluminate | 10 | 0.66 | 160/72.6 |
| Lithium Laurate Aluminate | 12 | 0.65 | 166/75.4 |
| Calcium Stearate Aluminate | 18 | 0.59 | 165/74.9 |
| Zinc Stearate Aluminate | 18 | 0.54 | 150/68.1 |
| Lithium Stearate Aluminate(LSA) | 18 | 0.55 | 180/81.7 |
| Lithium 12-OH-Stearate Aluminate | 18 | 0.68 | 150/68.1 |
| Lithium Behenate Aluminate | 22 | 0.73 | 175/79.5 |

*Average wear scar up to the break point.

In the above Tables VI and VII the listed compounds conform, essentially, to generic formula II or III as illustrated below by the approximate chemical formula shown.

| Trivialized Name* | Chemical Formula |
|---|---|
| Lithium Adipate Aluminate | $Li(C_6H_8O_4)_{0.5} \cdot 2Al(OH)_3$ |
| Lithium Decanoate Aluminate | $Li(C_{10}H_{19}O_2) \cdot 2Al(OH)_3$ |
| Lithium Laurate Aluminate | $Li(C_{12}H_{23}O_2) \cdot 2Al(OH)_3$ |
| Calcium Stearate Aluminate | $Ca_4(C_{18}H_{34}O_2)_4(SO_4)_2 \cdot 2Al(OH)_3$ |
| Zinc Stearate Aluminate | $Zn_4(C_{18}H_{34}O_2)_4(SO_4)_2 \cdot 2Al(OH)_3$ |
| Lithium Stearate Aluminate | $Li(C_{18}H_{35}O_2) \cdot 2Al(OH)_3$ |
| Lithium 12-HO-Stearate Aluminate | $Li(HO{-}C_{18}H_{34}O_2) \cdot 2Al(OH)_3$ |
| Lithium Behenate Aluminate | $Li(C_{22}H_{43}O_2) \cdot 2Al(OH)_3$ |

* Li compounds are formula I or II, Ca and Zn compounds are formula III.

Example 10

An aqueous solution of $AlCl_3$ was reacted with $NH_4OH$ thereby precipitating $Al(OH)_3$. The $Al(OH)_3$ was washed with $H_2O$ to wash out $NH_4Cl$ and a slurry of the $Al(OH)_3$ in water was reacted with LiOH at elevated temperature (95°C) to form crystalline $LiOH \cdot 2Al(OH)_3 \cdot nH_2O$.

A portion of the $LiOH \cdot 2Al(OH)_3 \cdot nH_2O$ slurried in water was titrated to pH=6 with $CCl_3COOH$ (trichloroacetic acid) to form crystalline $Li(CCl_3COO) \cdot 2Al(OH)_3 \cdot nH_2O$.

In a similar manner other lithium aluminates are prepared wherein the anion is $BO_2^-$, $NO_3^-$, $HCO_3^-$, $H_2PO_4^-$, $SO_4^{--}$, $F^-$, $CH_2ClCOO^-$, $CCl_2HCOO^-$, and the like.

X-ray diffraction patterns on the above products, and other products disclosed herein, indicate a crystalline material falling into the hexagonal crystal system with an interlayer distance of at least 7.5Å.

This distance is dependent on the size of the anion. These are 2- or 3-layer unit cell structures. The particle diameter is usually from 150Å to 10000Å. X-ray diffraction and scanning electron microscopic analysis have revealed its platelet structure. The ratio of the length to the thickness of these platelets can be between 1 and 1500. White powders or particles are generally produced, but tinted or colored products are not precluded from this invention.

The number of waters of hydration in the crystalline aluminates used in the present invention is generally within the range of 0 to 6.

Example 11

In one particular embodiment of the present invention, 73 g. of crystalline 3-layer $LiOH \cdot 2Al(OH)_3 \cdot nH_2O$ (wherein n is about 3) was dispersed in 400 ml. of drum grade 2-propanol and 114 g. of commercially available stearic acid (95 percent purity) was added. The mixture was stirred at 40°C for 1 hour, then filtered and the product analyzed. By analysis it is found that lithium stearate aluminate was formed, conforming to the formula $Li(RCOO) \cdot 2Al(OH)_3 \cdot nH_2O$ (wherein n is 0), the product also contained a small amount of unreacted stearic acid which can be substantially removed by washing with 2-propanol.

Example 12

The procedure of Example 11 was repeated using crystalline 2-layer $LiOH \cdot 2Al(OH)_3 \cdot nH_2O$ and substantially the same results were obtained except for the difference in the number of crystal layers.

Example 13

The procedure of Example 11 was repeated except that the amount of stearic acid used was less than enough to replace all the OH anions in the $LiOH \cdot 2Al(OH)_3 \cdot nH_2O$ crystal, and the product made conforms to the formula $Li(OH)_{1/2}(RCOO)_{1/2} \cdot 2Al(OH)_3 \cdot nH_2O$. This compound finds utility as an additive to organics and hydrocarbons, e.g. as a thickener, an acid ion scavenger, a viscosity-adjusting agent, a lubrication agent, as emulsion stabilizers, as solids dispersing agents, and the like.

**Claims**

1. A process for improving the anti-friction properties of lubrication fluids subjected to intensive shearing or grinding forces, which comprises

uniformly dispersing in said fluid, as small particles, at least one crystalline aluminate conforming substantially to the formula

$$\text{I. } (LiA_x)_y \cdot 2Al(OH)_3 \cdot nH_2O$$

$$\text{II. } Li(R)_r^v \cdot 2Al(OH)_3 \cdot nH_2O, \text{ or}$$

$$\text{III. } M_m(R)_r^v(D)_z^w \cdot 2Al(OH)_3 \cdot nH_2O$$

wherein A is one or more anions and/or negative-valence radicals or mixtures thereof;

wherein, in formula I, x is a quantity of A ions or radicals sufficient to substantially satisfy the valence requirements of Li;

wherein n is zero or the number of waters of hydration;

wherein y is a numerical value at least sufficient to maintain the crystalline structure;

wherein R is an anion of a monocarboxylic acid or dicarboxylic acid of $C_6$—$C_{22}$, including those which are OH— substituted;

wherein r is greater than zero and represents the number of R ions in the molecule;

wherein v is the valence of R, being monovalent or divalent;

wherein M is divalent Zn or Ca cations;

wherein D represents inorganic anions of valence 1-3, represented by w;

wherein z is equal to or greater than zero and represents the number of D anions;

wherein, in formula II, vr represents an amount of R anions to substantially satisfy the valence requirements of Li;

wherein, in formula III, r>z and (vr + wz) represents an amount of combined anions, R and D, to substantially satisfy the valence requirements of M;

with m representing the number of divalent M cations and having a numerical value in the range of 1 to 4.

2. The process of Claim 1 wherein the aluminate is at least one of formula I or II.

3. The process of Claim 1 wherein the amount of the aluminate dispersed in said lubrication fluid comprises 0.1 percent to 10 percent by weight.

4. The process of Claim 3 wherein the amount of the aluminate dispersed in said lubrication fluid comprises 0.2 percent to 2.0 percent by weight.

5. The process of Claim 2 wherein R is the anion of adipic acid, decanoic acid, lauric acid, hydroxystearic acid, stearic acid and behenic acid.

6. The process of Claim 1 wherein the aluminate is at least one of formula III.

7. The process of Claim 6 wherein the aluminate is at least one of $Ca_4(C_{18}H_{34}O_2)_4(SO_4)_2 \cdot 2Al(OH)_3$ or $Zn_4(C_{18}H_{34}O_2)_4(SO_4)_2 \cdot 2Al(OH)_3$.

8. A composition which comprises a substantially uniform mixture of a lubrication fluid and at least one compound of formula I as defined in Claim 1.

9. The composition of Claim 8 which comprises a mixture of a lubrication fluid and a crystalline lithium aluminate of the formula

$$IV.\ (Li(OH)_{1-gp}E_p^{-g})_y \cdot 2Al(OH)_3 \cdot nH_2O$$

wherein E is at least one anion or negative-valence radical other than $OH^-$, with a valence of $-g$;
wherein p is greater than or equal to zero, and is less than or equal to unity; and
wherein y and n are defined as in Claim 1.

**Patentansprüche**

1. Verfahren zur Verbesserung der Antifriktionseigenschaften von Schmierfluiden, welche intensiven Scher- oder Mahlkräften unterworfen sind, welches umfaßt:
gleichförmiges Dispergieren in diesem Fluid wenigstens eines kristallinen Aluminates als kleine Teilchen, welches im wesentlichen der Formel entspricht:

$$I.\ (LiA_x)_y \cdot 2Al(OH)_3 \cdot nH_2O$$

$$II.\ Li(R)_r^v \cdot 2Al(OH)_3 \cdot nH_2O,\ \text{oder}$$

$$III.\ M_m(R)_r^v(D)_z^w \cdot 2Al(OH)_3 \cdot nH_2O$$

worin A ein oder mehrere Anion/Anionen und/oder Rest/e mit negativer Wertigkeit oder Mischungen hiervon ist;

worin, in der Formel I, x eine Menge von A-Ionen oder Resten ist, um im wesentlichen die Wertigkeitserfordernisse des Li zu erfüllen;

worin n = 0 oder die Zahl des Hydratwassers ist;

worin y ein numerischer Wert ist, um wenigstens die kristalline Struktur aufrechtzuerhalten;

worin R ein Anion einer Monocarbonsäure oder Dicarbonsäure mit $C_{6-22}$, einschließlich solcher, die OH— substituiert sind, ist;

worin r größer als 0 ist und die Anzahl der R-Ionen in dem Molekül darstellt;

worin v die Wertigkeit von R, das einwertig oder zweiwertig sein kann, ist;

worin M zweiwertige Zn- oder Ca-Kationen darstellt;

worin D anorganische Anionen der Wertigkeit 1-3, die durch w dargestellt wird, bedeutet;

worin z gleich oder größer als 0 ist und die Anzahl der D-Anionen darstellt;

worin, in der Formel II, vr eine Menge an R-Anionen darstellt, um im wesentlichen die Wertigkeitserfordernisse des Li zu erfüllen;

worin, in Formel III, $r>z$ und $(vr + wz)$ eine Menge von kombinierten Anionen, R und D, darstellt, um im wesentlichen die Wertigkeitserfordernisse von M zu erfüllen;

wobei m die Anzahl an zweiwertigen M-Kationen darstellt und einen numerischen Wert im Bereich von 1 bis 4 hat.

2. Verfahren nach Anspruch 1, worin das Aluminat wenigstens eines der Formel I oder II ist.

3. Verfahren nach Anspruch 1, worin die Menge des in dem Schmierfluid dispergierten Aluminates 0,1 bis 10 Gew.-% umfaßt.

4. Verfahren nach Anspruch 3, worin die Menge des in dem Schmierfluid dispergierten Aluminates 0,2 bis 2,0 Gew.-% umfaßt.

5. Verfahren nach Anspruch 2, worin R das Anion von Adipinsäure, Decancarbonsäure, Laurinsäure, Hydroxystearinsäure, Stearinsäure und Behensäure ist.

6. Verfahren nach Anspruch 1, worin das Aluminat wenigstens eines der Formel III ist.

7. Verfahren nach Anspruch 6, worin das Aluminat wenigstens eines von

$$Ca_4(C_{18}H_{34}O_2)_4(SO_4)_2 \cdot 2Al(OH)_3$$

oder

$$Zn_4(C_{18}H_{34}O_2)_4(SO_4)_2 \cdot 2Al(OH)_3$$

ist.

8. Zusammensetzung, welche eine im wesentlichen gleichförmige Mischung eines Schmierfluids und wenigstens einer Verbindung der Formel I, wie in Anspruch 1 definiert, umfaßt.

9. Zusammensetzung nach Anspruch 8, welche eine Mischung von einem Schmierfluid und einem kristallinen Lithiumaluminat der Formel

$$IV.\ (Li(OH)_{1-gp}E_p^{-g})_y \cdot 2Al(OH)_3 \cdot nH_2O$$

umfaßt,

worin E wenigstens ein Anion oder Rest mit negativer Wertigkeit anders als $OH^-$ mit einer Wertigkeit von -g ist;

worin p größer oder gleich als 0 ist und kleiner als oder gleich 1 ist; und

worin y und n die in Anspruch 1 angegebenen Bedeutungen besitzen.

**Revendications**

1. Procédé pour améliorer les propriétés antifrottement de fluides lubrifiants soumis à des forces de cisaillement ou de meulage intensives, qui comprend l'opération de dispersion uniforme, dans ledit fluide, sous forme de petites particules, d'au moins un aluminate cristallin répondant essentiellement à la formule

$$I.\ (LiA_x)_y \cdot 2Al(OH)_3 \cdot nH_2O$$

$$II.\ Li(R)_r^v \cdot 2Al(OH)_3 \cdot nH_2O, \text{ ou}$$

$$III.\ M_m(R)_r^v(D)_z^w \cdot 2Al(OH)_3 \cdot nH_2O$$

où A est un ou plusieurs anions et/ou radicaux de valence négative ou des mélanges de ceux-ci;

où, dans la formule I, x est une quantité d'ions ou de radicaux A suffisante pour satisfaire essentiellement aux conditions de valence d Li;

où n est nul ou égal au nombre de molécules d'eau d'hydratation;

où y est une valeur numérique au moins suffisante pour maintenir la structure cristalline;

où R est un anion d'acide monocarboxylique ou d'acide dicarboxylique en $C_6$—$C_{22}$, y compris ceux qui sont substitués par —OH;

où r est supérieur à zéro et représente le nombre d'ions R dans la molécule;

oux v est la valence de R, qui est monovalent ou divalent;

où M est un cation Zn ou Ca divalent;

où D représente un anion minéral de valence 1—3, représenteé par w;

où z est supérieur ou égal à zéro et représente le nombre d'anions D;

où, dans la formule II, vr représente une quantité d'anions R permettant de satisfaire essentiellement aux conditions de valence de Li;

où, dans la formule III, r>z et (vr + wz) représente une quantité d'anions combinés, R et D, permettant de satisfaire essentiellement aux conditions de valence de M;

m représentant le nombre de cations M divalents et ayant une valeur numérique dans l'intervalle de 1 à 4.

2. Procédé selon la revendication 1, dans lequel l'aluminate est au moins un aluminate de formule I ou II.

3. Procédé selon la revendication 1, dans lequel la quantité d'aluminate dispersé dans ledit fluide lubrifiant constitue 0,1% à 10% en poids.

4. Procédé selon la revendication 3, dans lequel la quantité d'aluminate dispersé dans ledit fluide lubrifiant consitue 0,2% à 2,0% en poids.

5. Procédé selon la revendication 2, dans lequel R est l'anion de l'acide adipique, de l'acide décanoïque, de l'acide laurique, le l'acide hydroxystéarique, de l'acide stéarique ou de l'acide béhénique.

6. Procédé selon la revendication 1, dans lequel l'aluminate est au moins un aluminate de formule III.

7. Procédé selon la revendication 6, dans lequel l'aluminate est au moins l'un des aluminates

$$Ca_4(C_{18}H_{34}O_2)_4(SO_4)_2 \cdot 2Al(OH)_3 \text{ ou}$$

$$Zn_4(C_{18}H_{34}O_2)_4(SO_4)_2 \cdot 2Al(OH)_3.$$

8. Composition qui comprend un mélange essentiellement homogène d'un fluide lubrifiant et d'au moins un composé de formule I telle que définie dans la revendication 1.

9. Composition selon la revendication 8, qui comprend un mélange d'un fluide lubrifiant et d'un aluminate de lithium cristallin de formule

$$IV.\ (Li(OH)_{1-gp}E_p^{-g})_y \cdot 2Al(OH)_3 \cdot nH_2O$$

où E est au moins un anion ou un radical de valence négative autre que $OH^-$, avec une valence de -g;

où p est supérieur ou égal à zéro et est inférieur ou égal à l'unité; et

où y et n sont tels que définis dans la revendication 1.

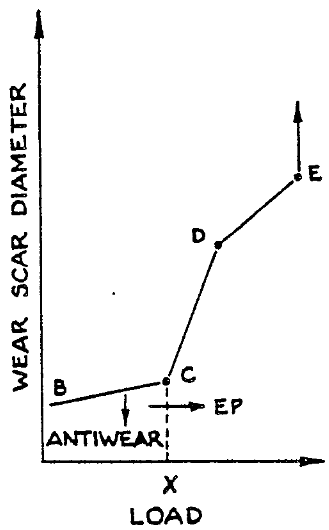


FIG. 1

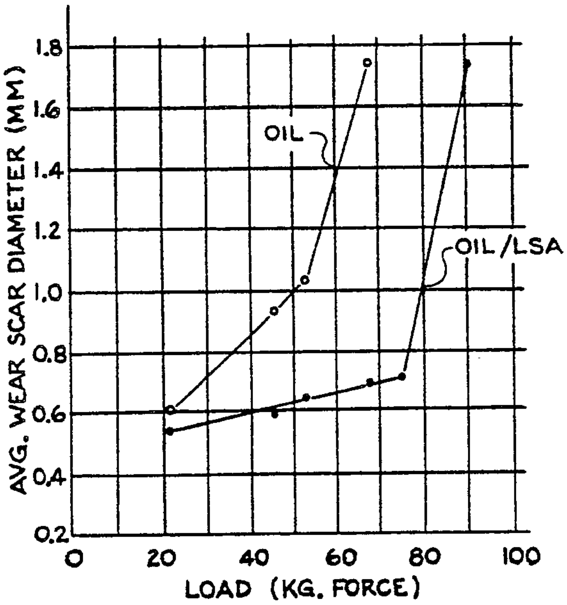


FIG. 2

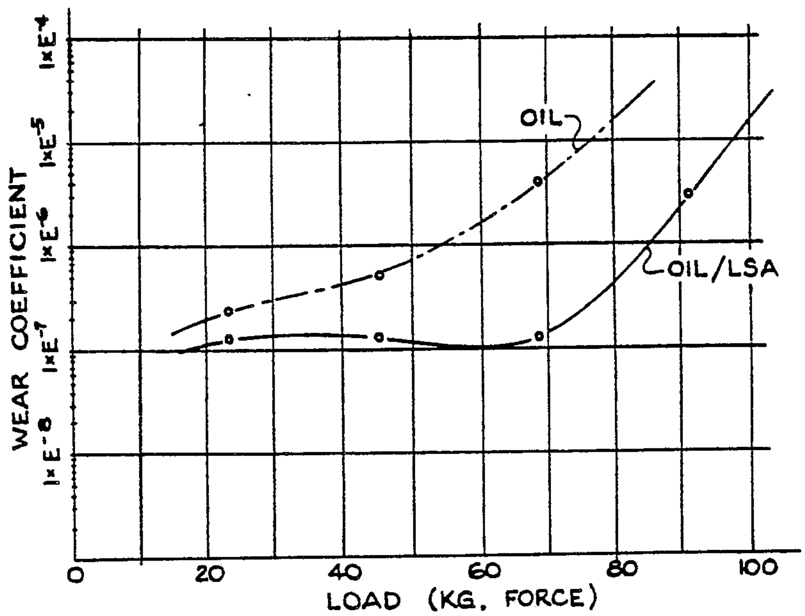


FIG. 3